# FASCICULE DE BREVET EUROPEEN

(11) **EP 0 708 836 B1**
(45) Date de publication et mention de la délivrance du brevet: **13.09.2000**
(21) Numéro de dépôt: 95918050.6
(22) Date de dépôt: 25.04.1995
(51) Int. Cl.: C12P 3/00, C05G 1/00, A61K 33/00

(54) **PROCEDE DE FABRICATION DE MATIERE MINERALE INACTIVEE, ET MATIERE INACTIVEE AINSI OBTENUE**
VERFAHREN ZUR HERSTELLUNG EINES INAKTIVIERTEN STOFFES UND SO ERHALTENER STOFF
METHOD FOR MAKING AN INACTIVATED MINERAL MATERIAL AND RESULTING INACTIVATED MATERIAL

(30) Priorité: 26.04.1994 FR 9405013; 21.04.1995 FR 9504831; 21.04.1995 FR 9504832; 21.04.1995 FR 9504830
(43) Date de publication de la demande: 01.05.1996
(73) Titulaire: SAVIEX, 75005 Paris (FR)
(72) Inventeur: Paradas, José, 95310 Saint-Ouen-L'Aumone (FR); Soleilhavoup, François, 93800 Epinay-sur-Seine (FR)
(74) Mandataire: Wagret, Frédéric
(86) Numéro de dépôt international: FR9500538
(87) Numéro de publication internationale: WO9529250

(56) Documents cités:
- EP-A- 0 388 304

## Description

La présente invention se rapporte à un procédé de fabrication ou production de matière minérale extemporanée et inactivée ainsi qu'à une matière inactivée obtenue suivant ce procédé.

La présente invention propose un procédé de production d'une matière minérale extemporanée, c'est-à-dire prête à être employée d'une manière quelconque. Par exemple, la matière ainsi produite pourra être utilisée comme "charge" ou apport dans un composé pour comblement, réagréage, cimentation, assemblage, apprêt, revêtement, hydrofugation, ou analogues.

Autrement dit, non seulement la matière conforme à l'invention se substitue à la pierre, mais celle-ci permet également d'envisager quantité d'applications inconnues à ce jour, dans des domaines aussi variés que la construction, la fabrication mécanique, électrique, phonique, l'agriculture, l'environnement, etc.

L'invention prévoit l'usage de toute "structure vivante" à pouvoir pétrifiant. Par "structure vivante" minéralisatrice, minéralisée ou minéralisable, il faut comprendre toute structure cellulaire ou d'origine cellulaire ( végétale, animale ou microorganique), vivante et/ou résultant de la vie et/ou des composés d'origine biologique, cristallisés ou non, tels des enzymes, hormones, protéines, ADN, etc.... Par "matière inactivée", il faut comprendre matière conforme à l'invention et dénuée de toute activité biologique et/ou biominéralisatrice notamment de toute activité microbiologique pathogène.

Les sources de matériau minéral disponibles pour la mise en oeuvre du procédé sont considérables. Le nombre de matériaux minéraux différents qu'il est possible d'utiliser, d'imiter ou de remplacer, par rapport à l'état de la technique, n'est limité que par l'état actuel de nos connaissances.

L'emploi de structures vivantes dans la technique propre à l'invention permet de faire appel à la récupération ou recyclage de déchets ou sous-produits constituant un apport en minéral ou aptes à générer un apport en minéral, en les valorisant.

A noter par ailleurs que l'invention s'avère économique quant à sa mise en pratique, et aboutit à un produit d'emploi suffisamment simple pour qu'il puisse être utilisé par tout un chacun, sans qualification, formation ou équipement spécialisé et coûteux.

Au vu de ce qui précède, l'invention a pour objet un procédé de fabrication ou obtention d'une matière ou composition minérale caractérisé par les étapes consistant à :
- préparer une base comprenant une quantité prédéterminée d'un matériau minéral ou bio minéral synthétisé par au moins une ou partie de structure vivante choisie parmi le règne végétal, animal et/ou les micro-organismes, ladite structure vivante étant cultivée durant une période et dans un milieu tels qu'au moins une partie dite 〈〈biomasse minérale〉〉 dudit matériau est alors produite ou synthétisée par cette structure; et
- traiter ladite base de façon à la transformer en une matière minérale ou bio minérale inactivée, notamment par une phase d'inactivation de la structure vivante, et de texture prédéfinie en fonction d'un usage extemporané choisi, par exemple en tant que charge d'apport, isolant thermique ou phonique, filtre, membrane acoustique, paroi décoratrice, tissu, fil, film, substance nutritive pour sols, substance à usage médical et/ou paramédical.

De préférence, la phase d'inactivation est effectuée par adjonction d'au moins un sel tel que par exemple l'oxyde de magnésium, le sulfate de magnésium, le chlorure de calcium, le chlorure de baryum, le fluosilicate de sodium ou le silicate de sodium, sous forme anhydre de préférence.

En outre, cette phase d'inactivation est exécutée suivant une ou plusieurs méthodes, seules ou en complément, dont l'irradiation, la déshydratation et/ou l'élévation à haute température.

D'une manière avantageuse, la structure vivante est sélectionnée pour que la matière minérale contienne au moins l'un des constituants suivants : précipités calciques, siliciques, fluorés, barytés, ferrugineux et autres.

Selon une forme particulière de réalisation, l'étape de préparation est réalisée in situ, par contact avec des micro-organismes et/ou des organismes minéralisateurs choisis dans un environnement adéquat.

Par ailleurs, l'invention concerne un substrat susceptible d'être inactivé obtenu suivant le procédé ci-dessus, à usage extemporané choisi, notamment comme isolant thermique, phonique, filtre, membrane acoustique, paroi décoratrice, tissu, fil, film.

Avantageusement, ce substrat est sous forme membranaire, souple ou solide, plus ou moins cristallisée, d'épaisseur variable, ou sous forme pulvérulente ou granuleuse, ou encore sous forme fluide, gazeuse ou liquide.

Selon un autre aspect, l'invention concerne un procédé de production d'une substance nutritive pour sols, naturels et/ou artificiels, caractérisé en ce que l'étape de préparation de la base comporte une phase consistant à doser celle-ci en éléments majeurs ou oligo-éléments suivant leurs insuffisances dans les sols et suivant les besoins des cultures.

Ce procédé permet d'obtenir une substance minérale nutritive inactivée, à usage extemporané, par exemple en tant que charge d'apport, notamment à tout type de sol naturel et/ou artificiel, ou encore toute composition du type engrais, terreau ou amendements divers.

D'autres méthodes d'inactivation peuvent être employées, seules ou en complément. dont l'irradiadon et l'élévation à une température supérieure à celle que la structure vivante supporte.

Une phase de malaxage peut être exécutée durant l'une des étapes du procédé.

De préférence, cette phase de malaxage est effectuée au moins en partie simultanément à une autre phase du procédé, et notamment de l'inactivation.

Le procédé selon l'invention est donc utilisable avec des microorganismes et/ou des cellules, d'origine végétale ou animale, seules, ou en association symbiotique ou autre, minéralisés, minéralisateurs ou minéralisables.

La structure vivante précitée peut être un végétal et/ou animal ou partie d'un végétal et/ou animal telle que cellule, tissu ou organe.

On a signalé, depuis fort longtemps, l'existence de nombreux composés de nature minéralogique variée et souvent complexe (calcique ou silicique par exemple), visibles à l'état figuré, dans les cellules ou d'origine cellulaire. Il s'agit de sels considérés jusqu'à maintenant comme des déchets de la vie cellulaire que l'organisme ne réemploie jamais. La présence de ces substances est généralement expliquée par le fait que l'organisme absorbe des sels (de calcium ou de silicium etc...) en quantité supérieure à ses besoins et qu'il se débarrasse du surplus sous forme cristallisée lors de réactions purement physico-chimiques.

Cependant, ces sels synthétisés dans l'organisme, ne résultent pas de simples réactions physicochimiques et des microorganismes minéralisateurs sont fréquemment impliqués dans ces dépôts de cellules végétales. Ces microorganismes minéralisateurs peuvent être multipliés à l'intérieur de ces cellules ou en être extraits et multipliés in vitro.

Ce phénomène, base du procédé de l'invention, est source de précipités non seulement calciques et/ou siliciques mais aussi fluorés, barytés, ferrugineux et autres. Il s'observe notamment dans tout le règne végétal, aussi bien chez les végétaux supérieurs que chez les végétaux inférieurs.

D'une façon générale ces dépôts peuvent contribuer à rigidifier les membranes et/ou les parois (Ils sont très abondants chez les végétaux inférieurs où ils incrustent les mycéliums cries parois des spores comme cela s'observe chez les Champignons Ascomycètes) et/ou intervenir dans la physiologie cellulaire.
On considère généralement que ces imprégnations (calcique et/ou silicique et/ou autres) sont uniquement sous la dépendance de la physiologie globale de l'organisme, ou d'organe(s) particulier(s). Or, les microorganismes minéralisateurs, présents (calcifiants, et/ou silicifiants et/ ou autres ) participent , par des échanges symbiotiques ou d'autre nature, à la minéralisation.

Le concept sur lequel repose le procédé de la présente invention réside en la présence et la participation effective, quasi générale, des microorganismes à pouvoir minéralisateur et entraîne des possibilités d'applications très variées.

On pourra ainsi avoir recours, dans le règne végétal, tout aussi bien à des végétaux supérieurs, dicotylédones, monocotylédones, qu'à des végétaux inférieurs thallophytiques ou microorganiques.

Selon les cas, la structure sélectionnée est cultivée in vivo, en terre, sur couche riche en madère organique, par hydroponie, en boîte de Pétri, dans un réacteur tel que fermenteur ou par élevage en batterie, sur pieds ou pisciculture, notamment.

Suivant une autre caractéristique, la structure est cultivée dans un milieu nutritif approprié connu de l'homme de l'art tel que liquide de Knopp, solution d'Earle, Hanks, milieu dit "199", milieu de Sabouraud, milieu MEM-Eagle ou analogues.

Suivant encore une autre caractéristique, l'étape de préparation de la base comporte une phase consistant à récolter et/ou collecter la structure vivante précitée, et à l'incorporer à la base dans des proportions telles que cet apport constitue au moins une partie du matériau minéral requis.

Le matériau minéral ainsi récolté et/ou collecté pourra être de nature variée: carbonatée, siliceuse, saline, fluorée, baryrée, carbonée, ferrugineuse, sous forme par exemple de dépôt, concrétionnement actuels et/ou fossiles. Il est alors incorporé à la base soit lors de l'étape de préparation, soit lors de l'étape de traitement.

Mais des roches sédimentaires fossiles et/ou autres telles que granite, basalte, pierre ponce ou autres peuvent entrer dans la composition de la matière finale. Durant une phase possible du procédé, au moins l'un parmi la biomasse, la base , le matériau et/ou la matière minérale précités est fragmenté lors de l'étape de préparation, et/ou lors de l'étape de traitement.

L'invention est caractérisée en ce qu'en outre, la phase de fragmentation est effectuée jusqu'à obtention d'une texture ou malléabilité déterminée.

La fragmentation précitée est au moins partiellement effectuée par dislocation à l'aide d'ultrasons et/ou de moyens physico-chimiques, tels qu'adjontion d'additifs, irradiation, traitement cryogénique et/ou thermique, broyage ou variation de pression.

L'une des phases du procédé peut consister à élaborer une suspension de la biomasse, de la base ou du matériau précités dans un liquide, de préférence aqueux pour pulvérisation ou application au pinceau par exemple. Ce liquide peut entrer dans la composition de l'un des milieux de culture précités.

Il est possible d'incorporer lors d'une phase du procédé, au moins à l'un parmi le milieu de culture, la biomasse, le matériau, la base et la matière minérale, une substance colorante de nuance prédéterminée.

Une phase éventuelle de l'étape de préparation et/ou de traitement consiste à incorporer à l'un au moins parmi la biomasse, le milieu, le matériau, la base et la matière minérale, un agent de cohésion et/ou de texture.

Cet agent de cohésion et/ou de texture est de préférence métallique tel que calcium, magnésium, silicium, baryum, sodium, fluor, aluminium, fer, manganèse, zinc, ou organique tel que collagène, muco-polysaccharide et/ou composé poly-cellulosique.

Avantageusement, les proportions et la composition de l'agent de texture précité sont choisies pour que la matière finale présente une dureté et/ou élasticité prédéterminée.

Par ailleurs, le procédé est caractérisé en ce qu'il comprend une phase de déshydratation totale ou partielle du ou des sels d'inactivation composant la base et/ou de la matière minérale précitées.

Cette phase de déshydratation peut être au moins partiellement effectuée par filtrage, centrifugation, traitement thermique et/ou cryogénique etc....

Il est envisageable suivant le procédé, que les phases d'inactivation, déshydratation et fragmentation, soient au moins partiellement effectuées simultanément.

Durant l'une des phases ou étapes du procédé, un additif par exemple moussant, fibreux, agglomérant, isolant, ignifuge ou analogues peut être incorporé à la base et/ou à la matière minérale précitées.

L'étape de traitement peut comprendre une phase de conditionnement dans un emballage approprié notamment en aérosol, de la matière minérale qui constitue un produit fini.

L'invention a d'autre part pour objet une matière ou composition minérale inactivée à usage extemporané, et par exemple en tant que charge d'apport, sous forme solide, pâteuse ou fluide, cette matière étant obtenue suivant le procédé expliqué ci-dessus.

Mais d'autres avantages et particularités de l'invention ressortiront mieux de la description détaillée d'exemples de sa mise en pratique, et qui sont expliqués ci-après.

Le procédé de fabrication ou obtention d'une matière ou composition minérale inactivée à usage extemporané comprend principalement deux étapes. La première consiste à préparer une base comprenant une quantité prédéterminée d'un matériau minéral synthétisé par au moins une ou partie de structure vivante sélectionnée parmi le règne végétal, animal et/ou les micro-organismes.

La classification ci-après donne des exemples non limitatifs de structures aptes à être sélectionnées pour l'application de l'invention:
a)- Parmi les Dicotylédones:
   - Les cellules de l'ordre des Daucales, famille des Araliacées, genre Hedera dont Hedera helix;
   - Les cellules de l'ordre des Arales, genre Rhaphidophora, espèce Syngonium podophyllum (Schott), "Albolineatum", espèces Syngonium auritum syn. Philodendron trifolium et espèce Syngonium hastifolium:
   - Les cellules de l'ordre des Solanales, famille des Bignoniacées, espèce Catalpa bignonloidées où il s'agit d'oxalate de calcium sous forme octaédrique, "en oursins", présents en abondance dans les cellules des pétioles, par exemple.

   Mais, de surcroît, toute cellule végétale, même celle qui serait peu symbiotique est susceptible, par le procédé que nous venons d'énoncer. d'être minéralisée et/ou surminéralisée si on la met en contact avec de tels microorganismes.
   C'est ainsi le cas des cellules précitées, mais aussi de toutes les cellules végétales issues par exemple de broussailles sous forme de copeaux, de sciure, et qui par ce procédé se pétrifient = se transforment en pierre tuffeuse et/ou travertineuse.
b)- Parmi les Monocotylédones:
   - Les cellules de l'ordre des Pandarales dont celles de la famille des Typhacées;
   - Les cellules de graminées dont celles des Lemnacées,
c)- Parmi les Fougères:
   Les épidermes chez Equisetum arvense (Prêle commune) sont le théâtre de productions minérales de silice souvent considérés comme des concrétions issues, de sécrétions membranaires épidermiques alors que s'y trouvent impliquées des bactéries en périphérie et/ou dans les cellules d'Equisetum arvense. Celles-ci expliquent les dépôts de silice intra et/ou extracellulaire.
   Le recouvrement siliceux de la pousse aérienne stérile de l'Equiseturn arvense et les dépôts de silice opaline incrustant l'épiderme de ses différents organes aériens est lié à la présence et la participation symbiotique de bactéries. Il s'agit de bacilles de 0,4 à 0,7 microns de longueur et de 0,1 micron d'épaisseur. Ces bacilles minéralisateurs provoquent un empâtement externe progressif des cellules intéressées au fur et à mesure du développement des voiles bactériens. Ces voiles successifs provoque un recouvrement siliceux homogène et stratifié.
d)- Parmi les Algues:
   Des masses biominérales résultant de la multiplication et de l'accumulation de frustules d'algues de façon naturelle ou industrielle sont applicables à l'invention. On citera par exemple:
   - Les Rhodophycées avec principalement les Némalionales, les Solénoporacées et les Corallinacées (comportant les Lithothamnium, les Mélobésiées;
   - Les Chlorophycées avec les Siphonales (comportant les Codiacées dont Halimeda) et les Dacycladales (comportant les dacycladacées dont Acétabularia);
   - Les Charophycées avec les characées dont Cladophora et Vaucheria;
   - Les Schizophycées avec les Porostromata et les Spongiostroma dont Rivularia, Oscillatoria, Phormidium, Chroococcus et Gleocapsa sont aptes à être sélectionnés.
e)- Des Lichens:
   La masse biominérale peut aussi résulter de lu culture d'une association végétale tripartite de type lichénique. Les Lichens sont des associations végétales composites. L'association dite à bénéfice réciproque se compose d'un champignon, d'une algue et de bactéries minéralisantes semblables à celles dont nous avons fait état plus haut. Ces bactéries contribuent à des incrustations minérales complexes et pigmentées au sein du mycélium.
   L'existence de dépôts d'acides lichéniques sous forme de cristaux extracellulaires et hydrophobes dûs à la minéralisation des lichens par lesdites bactéries symbiotes. Les bactéries symbiotes se distinguent des cyanobactéries parfois citées par leur petite taille et l'absence de pigment.
   Dans la présente invention la structure vivante peut donc être tout simplement les cellules d'un végétal ainsi physiologiquement équipé.
f)-Parmi les Champignons:
   La structure d'origine végétale peut être un végétal inférieur choisi parmi les champignons et leurs spores.
g)-Parmi les microorganismes:
   Evidemment, la structure vivante peut être un micro-organisme tel que virus ou bactérie dont bacillus mégathérium, pseudomonas fluorescens, pseudomonas maltophilia, pseudomonas putida, buttauxiella agrestis, rhodococcus, serratia marescens.
   On peut envisager l'emploi d'un virus en tant que structure vivante.

La structure vivante peut aussi être un animal, ou partie d'animal telle que cellule, tissu et/ou organe, choisi parmi les protozoaires ou les métazoaires invertébrés tels les spongiaires, les lamellibranches et les échinodermes ou encore parmi les vertébrés.

Il va de soi qu'une ou plusieurs structures vivantes compatibles, symbiotiques ou coopérantes peuvent être cultivées au sein d'un même milieu, qu'elles soient d'origine végétale, animale et/ou micro-organique.

La première étape de préparation a pour but d'obtenir une base minérale, c'est-à-dire un produit intermédiaire. De préférence, l'étape de préparation de la base comporte une phase consistant à cultiver la structure vivante précitée durant une période et dans un milieu tels qu'au moins une partie dite "biomasse minérale" dudit matériau est alors
produite ou synthétisée par cette structure.

La seconde étape consiste à traiter ladite base après sa surminéralisation de façon à la transformer en une matière minérale désactivée ou inactivée et de texture prédéfinie en fonction d'un usage extemporané choisi.

A titre d'exemple, l'une ou plusieurs des correspondances entre les structures vivantes et les minéraux et/ou les roches ci-après, peuvent être sélectionnés conformément à l'invention:
Cellules de Hedera helix ↔ Oxalate de calcium/ Calcaire
Cellules de Ficus élastica ↔ Carbonate de calcium/ Calcaire
Cellules d'Equisetum ↔ Silice / Opaline / Bois silicifié / Grès
Cellules de Graminées ↔ Silice / Opaline / Bois silicifié / Grès
Cellules de Typhacées ↔ Silice / Opaline / Bois silicifié / Grès
Champignon Ascomycète ↔ Dipicolinate de calcium/ Calcaire
Cellules de Pectascinacées ↔ Carbonate de calcium/ Calcaire
Algue Phéophysée ↔ Silice / Diatomite /
Algue Rhodophycée, ↔ Carbonate de calcium/ Calcaire / Travertin
Algue Chlorophycée, ↔ Carbonate de calcium / Calcaire / Travertin
Algue Cyanophycée ↔ Carbonare de calcium / Calcaire / Travertin
Mollusque Lamellibranche ↔ Carbonate de calcium / Calcaire / Grès coquilliers

La production de biomasse obéit aux lois usuelles de la croissance des structures vivantes: il n'est pas nécessaire de décrire ici la phase de culture avec précision. L'homme de l'art saura déterminer en fonction de la structure vivante à faire croître, quelle méthode de culture et quel milieu pourront être utilisés avec le plus de succès.

Ainsi, il est possible selon le type de structure sélectionné, de recourir à l'un des procédés élaborés tels que la culture in vivo, en terre, sur couche riche en matière organique, par hydroponie, en boîte de Pétri, dans un réacteur tel que fermenteur ou par élevage en batterie, sur pieds ou pisciculture, notamment.

Dans le cas des plantes conservant leur intégrité, les méthodes conventionnelles de culture sont de préférence employées (en terre, en serre, hydroponie, etc).

Lorsque la structure vivante précitée est une partie d'un végétal telle que cellule, tissu ou organe, la structure vivante peut donc avoir pour origine une plante dite supérieure, monocotylédone, comme les Lentilles d'eau ou dicotylédone et notamment parmi les Daucales, les Lianes, les Bignoniacées, les Mortes, les Cornacées, les Cactacées.

Pour ce type de structure qui, par exemple produit une matière tuffeuse et/ou travertineuse, il est possible d'utiliser le milieu de culture suivant:

| | |
|---|---|
| Eau distillée | 1 000 grammes |
| Nitrate de calcium | 0,71 grammes |
| Nitrate de potassium | 0,568 grammes |
| Sulfate de magnésium | 0,284 grammes |
| Phosphate d'ammonium | 0,142 grammes |
| Chlorure ferrique | 0,112 grammes |
| Iodure de potassium | 0,0028 grammes |
| Acide borique | 0,0005 grammes |
| Sulfate de Zinc | 0,0005 grammes |
| Sulfate de manganèse | 0,0005 grammes |

Mais, par ailleurs, la structure d'origine végétale peut être une algue et notamment Rhodophycée, Chlorophycée, Charophycée, Schyzophycée, Cyanophycée, Pheophycée et/ou encore un Protophyte, et alors d'autres milieux sont envisageables.

Il en va de même si la structure vivante est un animal, ou partie d'animal telle que cellule, tissu et/ou organe, et notamment un protozoaire à test arénacé, carbonaté, siliceux ou chitineux, des mesoglées de spongiaires aux spicules calcaires ou siliceux, des cellules de coralliaires, des cellules épidermiques de coquillages, ou encore des cellules à l'origine de structures osseuses chez les vertébrés. Les biomasses minérales pourront être obtenues par culture in-vitro des cellules tissu et/ou organe impliqués dans les biosynchèses minérales du règne animal.

Il est possible de recourir par exemple à des productions coralliaires actuelles et/ou fossiles pour réaliser des calcaires spécifiques.

Les micro-organismes sont également cultivables de façon abondante dans des fermenteurs industriels du commerce, et notamment des ultra-fermenteurs à filtrage tangentiel, qui permettent d'obtenir une production en continu.

Une ou plusieurs structures vivantes compatibles peuvent être cultivées au sein d'un même milieu, éventuellement avec au moins une autre structure compatible d'origine végétale, animale et/ou microorganique.

La seconde étape consiste à traiter ladite base après su surminéralisation de façon à la transformer en une matière minérale inacrivée et de texture prédéfinie en fonction d'un usage extemporané choisi.

En cas de production de biomasse à partir d'une structure vivante, l'étape du traitement comprend alors une phase d'inactivation de la structure vivante.

Cette phase d'inactivation peut être effectuée par adjonction d'au moins un sel tel que par exemple oxyde de magnesium, sulfate de magnésium, chlorure de calcium, chlorure de baryum sous forme anhydre de préférence.

La biomasse est stabilisée, c'est-à-dire inactivée de façon que tout développement cellulaire soit interrompu. Un tel résultat peut être obtenu par ajout dans des proportions convenables, de l'un ou plusieurs des sels ci-dessus, ou de substances ayant des effets analogues sur la structure vivante retenue.

Dans le cas de l'utilisation de ces composés, les proportions (en unité de masse) respectives de 1 partie de sulfate de magnésium, 0.5 partie de chlorure de calcium, 0.5 partie de chlorure de baryum, 2 parties d'oxyde de magnésium de préférence anhydres, sont acceptables. Toutefois, ces proportions peuvent varier dans une gamme de 20% à 50%.

D'autres méthodes d'inactivation peuvent être employées, seules ou en complément, dont l'utilisation de composés chimiques dont notamment des fluosilicates ou encore l'irradiation et l'élévation à haute température. Une phase de malaxage peut être exécutée durant l'une des étapes du procédé. De préférence, cette phase de malaxage est effectuée au moins en partie simultanément à une autre phase du procédé, et notamment lors de l'inactivation.

On peut recourir à deux phases d'inactivation: l'une lors de l'élaboration du produit, l'autre lors de l'emploi. L'inactivation totale des structures vivantes de la base se réalise alors par malaxage de la biomasse et des sels cités, avec un apport d'eau en quantité égale, pour garantir un bon mélange de ceux-ci. A titre d'exemple, on peut ajouter la base aux sels cités dans des proportions allant de 0.5 de base pour 1 de sels à 4 de base pour 1 de sels.

L'un des nombreux avantages de l'invention consiste en ce que l'étape de préparation de la base peut comporter une phase consistant à récolter ou collecter soit la structure vivante précitée soit du matériau minéral sédimentaire, et à l'incorporer à la base dans des proportions telles que cet apport constitue au moins une partie du matériau minéral requis.

Il est possible de collecter le matériau minéral sous forme par exemple de dépôt, concrétionnement actuel et/ou fossile, et de l'incorporer à la base soit lors de l'étape de préparation, soit lors de l'étape de traitement.

Par ailleurs, la structure vivante est choisie pour que sa matière minérale associée contienne au moins un constituant minéralogique carbonaté, siliceux, séléniteux ou analogues.

De nombreuses autres sources nouvelles de minéral sont utilisables conformémment à l'invention. Le débrousaillage ou d'autres opérations agricoles (récoltes dont fenaison, moisson, vendanges et autres) génèrent d'importantes quantités de produits minéralisés ou minéralisables ou minéralisateurs et aptes à une sur-minéralisation (foin, paille, chaume etc...).

Certaines opérations industrielles génèrent des déchets d'origine végétale, telle la sciure, pouvant être minéralisés. On cite les cellules des plantes relativement rigides et particulièrement celles des Lianes comme par exemple Syngonium podophyllum, Syngonium auritum syn. Philodendron trifolium et Syngonium hastifolium. Des Lierres, des Figuiers, constituent une source potentielle de substrats minéraux. Il en va de même pour les dépôts côtiers d'algues marines tels que le Maerl.

Durant une phase possible du procédé, au moins l'un parmi la biomasse, la base, le matériau et/ou la matière minérale précités est fragmenté lors de l'étape de préparation, et éventuellement lors de l'étape de traitement inactivant.

Par exemple si lors de la culture d'une structure vivante on obtient des empilements minéraux et/ou stratifiés, qu'on peut éventuellement morceler pour leur emploi au sein de la base. De même, si une partie ou la totalité du matériau minéral est constitué par des fossiles ou déchets solides, comme expliqué plus haut, les blocs en masses collectés pourront être broyés ou fragmentés.

La phase de fragmentation est effectuée jusqu'à obtention d'une texture ou malléabilité déterminée. Autrement dit, la fragmentation a pour but d'obtenir une matière plus ou moins meuble, et de préférence avec une granulométrie donnée.

La fragmentation précitée est au moins partiellement effectuée par dislocation à l'aide d'ultrasons et/ou de moyens physico-chimiques, tels qu'adjontion d'additifs, traitement cryogénique et/ou thermique, broyage ou variation de pression.

Dans ce cas, l'une des phases du procédé peut consister à élaborer une suspension de la biomasse ou du matériau fragmentés précités dans un liquide, de préférence aqueux.

Il est possible d'incorporer lors d'une phase du procédé, au moins à l'un parmi le milieu de culture, la biomasse, le matériau, la base et la matière minérale, une substance colorante de nuance prédéterminée. Cette substance est éventuellement un élément de la biomasse.

Une étape de préparation et/ou de traitement peut consister à incorporer à l'un au moins parmi la biomasse, le milieu, le matériau, la base et la matière minérale, un agent de cohésion ou texture. Cet agent est de préférence un liant notamment métallique tel que calcium, magnésium, silicium, baryum, sodium, fluor, aluminium, fer, manganèse, zinc, ou organique tel que collagène, muco-polysaccharide et composé poly-cellulosique.
On comprend déjà que cet agent fait éventuellement office de composé d'inactivation, dans le cas d'une base comportant une structure vivante, cultivée ou collectée.

Avantageusement, les proportions et la composition de l'agent de cohésion et/ou de texture précité sont choisies pour que la matière finale présente une dureté ou élasticité prédéterminée.

Durant l'une des phases ou étapes du procédé, un additif par exemple moussant, fibreux, agglomérant, isolant, ignifuge ou analogues peut être incorporé à la base et/ou à la matière minérale précitées.

La composition de la matière minérale ou biominérale peut être choisie en fonction du substrat sur lequel elle doit être appliquée. On pourra ainsi imiter la "patine" d'un matériau quelconque, ou reconstituer l'agencement pétrographique de toute surface artificielle.

Par ailleurs, le procédé est caractérisé en ce qu'il comprend une phase de déshydratation au moins partielle d'un ou plusieurs composants de la base et/ou de la matière minérale précitées.

Cette phase de déshydratation peut être au moins partiellement effectuée par filtrage, irradiation, centrifugation, traitement thermique et/ou cryogénique.

Il est envisageable suivant le procédé, que les phases d'inactivation, déshydratation et fragmentation, soient au moins partiellement effectuées simultanément.

L'étape de traitement peut comprendre une phase de conditionnement dans un emballage approprié, de la matière minérale qui constitue un produit fini.

Un exemple de déroulement des phases du procédé peut être envisagé comme suit: la base est constituée par une biomasse obtenue par culture in vitro d'un microorganisme minéralisateur en présence de cellules végétales résiduelles issues du broyat de broussailles, copeaux, sciure etc... La suspension biominérale obtenue est colorée puis séchée et le mélange est alors malaxé et subit un apport de sels de inactivation. Cette poudre est utilisable comme un ciment, et se durcit après séchage.

Le procédé décrit dans la présente demande, selon l'invention concerne également un procédé de production de substance nutritive des sols, naturels et/ou artificiels, par biominéralisation, ainsi que la substance nutritive obtenue par ce procédé utilisable dans les domaines de l'agronomie, l'agriculture, l'horticulture et des industries découlant de ces activités telles la production des engrais et divers amendements.

Les sols présentent des déficiences structurales et/ou des carences chimiques qui limitent qualitativement et quantitativement les produits récoltés; ils sont souvent trop calcaires, trop siliceux ou encore trop argileux et ne répondent jamais exactement aux besoins des plantes cultivées. Il en résulte des phénomènes de dessiccation et/ou de compaction, ou à l'inverse, des phénomènes d'engorgements hydriques voire d'hydromorphie.

Les procédés connus pour tenter de prévenir de tels phénomènes consistent à réaliser un apport en calcaire ou en chaux afin de remédier à l'excès d'acidité et/ou à un défaut de structure d'une terre agricole. Cette technique connue compense plus ou moins et souvent à court terme, les déséquilibres patents des sols, mais s'avère être grossière lors d'une application prolongée. En effet, l'apport répété, empirique et aveugle (irrégularités des épandages; doses inadaptées aux besoins réels des sols) d'éléments étrangers à la composition initiale d'un sol pallie imparfaitement les déficiences structurales et/ou les carences chimiques et peut entraîner des désordres en tout genre, notamment à long terme.

Les traitements par intrants chimiques intensifs s'avèrent coûteux et polluants : le calcaire peut bloquer en les rendant insolubles certains éléments indispensables aux plantes comme le fer et divers oligoéléments (manganèse, zinc, cuivre) provoquant ainsi des carences.

La présente invention remédie à ces inconvénients et propose un procédé de production d'une substance nutritive pour sols naturels et/ou artificiels. Ce procédé améliore l'activité micro-organique du sol, accélère l'humification et la minéralisation tout en neutralisant les ions toxiques. L'amélioration des principales propriétés chimiques du sol est rapide et importante : la décomposition de la matière organique est accélérée, ce qui facilite l'intégration de celle-ci au sol. Le procédé offre des possibilités de fertilisation localisée selon la variabilité des sols au sein d'une même parcelle, améliore le drainage ou encore le compactage et/ou le décompactage de la terre. Dans le cas d'une substance nutritive biominérale calcique, le procédé offre un calcaire actif d'un degré de finesse comparable à celui des meilleurs limons qui favorise notamment la floculation de l'humus. Ce procédé de production est caractérisé par les étapes consistant à :
- préparer une base comprenant une quantité prédéterminée d'un matériau minéral synthétisé par au moins une ou partie de structure vivante choisie parmi le règne végétal, animal et/ou les micro-organismes ; et
- traiter ladite base de façon à la transformer en une substance minérale inactivée nutritive et de texture prédéfinie selon la nature et les besoins des sols concernés afin d'améliorer à la fois la structure et la composition biochimique et/ou biologique des sols naturels et/ou artificiels destinés à la culture.

L'invention peut faire appel à toute "structure vivante" à pouvoir pétrifiant. Par "structure vivante", minéralisatrice, minéralisée ou minéralisable, il faut comprendre toute structure cellulaire ou d'origine cellulaire (végétale, animale ou micro-organique) vivante et/ou résultant de la vie et/ou des composés d'origine biologique, cristallisés ou non, tels des enzymes, hormones, protéines, ADN, etc.

Par "matière inactivée", il faut comprendre matière conforme à l'invention et dénuée de toute activité biologique et/ou biominéralisatrice, notamment de toute activité micro-biologique pathogène.

Les sources de matériau minéral disponibles pour la mise en oeuvre du procédé de l'invention sont considérables, d'autant qu'il est possible d'employer des structures vivantes provenant de récupération ou recyclage de déchets ou sous-produits constituant un apport en minéral ou aptes à engendrer un apport en mineral, en les valorisant.

A noter, par ailleurs, que l'invention s'avère économique quant à sa mise en pratique et aboutit à un produit d'emploi suffisamment simple pour qu'il puisse être utilisé par tout un chacun, sans qualification, formation ou équipement spécialisés et coûteux.

Un des nombreux avantages de l'invention consiste à doser, dans l'étape de préparation de la base, celle-ci en eléments majeurs et/ou oligo-éléments suivant leurs insuffisances dans les sols ou suivant les besoins des cultures. Cette phase de dosage permet avantageusement de traiter tous sols (acides ou basiques) en réalisant un apport spécifique (calcium, silice, ou autres suivant les besoins).

Un autre avantage de l'invention consiste en l'emploi de micro-organismes et/ou des organismes minéralisateurs choisis dans un environnement adéquat. La biominéralisation s'intègre donc au cycle naturel des sols contrairement à la technique connue de chaulage et/ou d'épandage d'engrais classique, tels les super phosphates, etc.

La présente invention concerne également la substance nutritive biominéralisée obtenue par le procédé précédent et prête à être employée dans les domaines aussi variés que ceux relevant des activités agronomiques, agricoles et horticoles et des industries découlant de ces activités telles la production des engrais et divers amendements. Elle présente l'avantage de fournir un apport de structure poreuse aux sols traités, favorisant les échanges gazeux et la circulation de l'eau à l'intérieur du sol et entre le sol et l'atmosphère ambiante.

D'autres avantages et particularités de l'invention ressortiront mieux de la description détaillée d'exemples de sa mise en pratique, et qui sont expliqués ci-après.

Le procédé de fabrication ou obtention d'une matière ou composition minérale inactivée à usage extemporané comprend principalement deux étapes.

La première étape consiste à préparer une base comprenant une quantité prédéterminée d'un matériau minéral synthétisé par au moins une ou partie de structure vivante sélectionnée parmi le règne végétal, animal et/ou les micro-organismes. Cette base minérale constitue un produit intermédiaire.

La classification ci-après donne des exemples non limitatifs de structures vivantes aptes à être sélectionnées pour l'application de l'invention:
a) - Parmi les Dicotylédones:
   - Les cellules de l'ordre des Daucales, famille des Araliacées, genre Hedera dont Hedera helix ;
   - Les cellules de l'ordre des Arales, genre Rhaphidophora, espèce Syngonium podophyllum (Schott), "Albolineatum", espèces Syngonium auritum syn. Philodendron trifolium et espèce Syngonium hastifolium ;
   - Les cellules de l'ordre des Solanales, famille des Bignoniacées, espèce Catalpa bignonioidées.

   De surcroît, toute cellule végétale, même celle qui serait peu symbiotique est susceptible, par le procédé de l'invention, d'être minéralisée et/ou surminéralisée si on la met en contact avec de tels microorganismes.
   C'est ainsi le cas des cellules précitées, mais aussi de toutes les cellules végétales issues par exemple de broussailles sous forme de copeaux, de sciure, et qui par ce procédé se pétrifient c'est-à-dire se transforment en substrat pétrifié, carbonaté et/ou silico-carbonaté.
b) - Parmi les Monocotylédones :
   - Les cellules de l'ordre des Pandarales dont celles de la famille des Typhacées ;
   - Les cellules de graminées dont celles des Lemnacées.
c)- Parmi les Fougères :
   Les épidermes chez Equisetum arvense (Prêle commune) sont le théâtre de produc:ions minérales de silice souvent considérés comme des concrétions issues, de sécrétions membranaires épidermiques alors que s'y trouvent impliquées des bactéries en périphérie et/ou dans les cellules d'Equisetum arvense. Celles-ci expliquent les dépôts de silice intra et/ou exeracellulaire.
d) - Parmi les Algues:
   Des masses biominérales résultant de la multiplication et de l'accumulation de frustules d'algues de façon naturelle ou industrielle sont applicables à l'invention. Par exemple
   - Les Rhodophytes avec principalement les Némalionales, les Solénoporacées et les Corallinacées (comportant les Lithothamnium, les Mélobésiées);
   - Les Chromophytes qui réunissent notamment les Chromophycées et surtout les Diatomophycées aux productions siliceuses;
   - Les Chlorophytes avec les Siphonales (comportant les Codiacées dont Halimeda) et les Dacycladales (comportant les dacycladacées dont Acétabularia) ;
   - Les Cyanophytes avec les Porostromata et les Spongiostrorna dont Rivularia, Oscillatoria, Phormidium, Chroococcus et Gleocapsa sont aptes à être sélectionnés.
e) - Des Lichens :
   La masse biominérale peut aussi résulter de la culture d'une association végétale tripartite de type lichénique. Les Lichens sont des associations végétales composites. L'association dite à bénéfice réciproque se compose d'un champignon, d'une algue et de bactéries minéralisantes, dites bactéries symbiotes, semblables à celles visées précédemment. Ces bactéries contribuent à des incrustations minérales complexes et pigmentées au sein du mycélium.
f) - Parmi les Champignons et leurs spores.
g) - Parmi les microorganisines : virus ou bactérie dont bacillus mégathérium, pseudomonas fluorescens, pseudomonas maltophilia, pseudomonas putida, buttauxiella agrestis, rhodococcus, serratia marescens.

La structure vivante peut aussi être un animal, ou partie d'animal telle que cellule, tissu et/ou organe, choisi parmi les protozoaires ou les métazoaires invertébrés tels les spongiaires, les lamellibranches et les échinodermes ou encore parmi les vertébrés.

De préférence, l'étape de préparation de la base comporte une phase consistant à cultiver la structure vivante précitée durant une période et dans un milieu tels qu'au moins une partie dite "biomasse minérale" dudit matériau est alors produite ou synthétisée par cette structure.

A titre d'exemple, l'une ou plusieurs des correspondances entre les structures vivantes et les minéraux et/ou les roches ci-après, peuvent être sélectionnés conformément à l'invention:
Cellules de Hedera helix ↔ Oxalate de calcium/Calcaire
Cellules de Ficus élastica ↔ Carbonate de calcium/ Calcaire
Cellules d'Equisetum ↔ Silice/Opaline/Bois silicifié /Grès
Cellules de Graminées <--> Silice / Opaline / Bois silicifié /Grès
Cellules de Typhacées <--> Silice / Opaline / Bois silicifié /Grès
Champignon Ascomycète <--> Dipicolinate de calcium/ Calcaire
Cellules de Pectascinacées ↔ Carbonate de calcium/ Calcaire
Algue Phéophysée ↔ Silice / Diatomite /
Algue Rhodophycée, ↔ Carbonate de calcium / Calcaire / Travertin
Algue Chlorophycée, ↔ Carbonate de calcium / Calcaire / Travertin
Algue Cyanophycée ↔ Carbonate de calcium / Calcaire / Travertin
Mollusque Lamellibranche <--> Carbonate de calcium / Calcaire /Grès coquilliers

Le procédé de l'invention permet ainsi de produire des substances carbonatées en vue du traitement des sols acides, grâce à l'apport de carbonate de calcium d'origine biologique et des substances siliceuses en vue du traitement des sols basiques grâce à un apport de silice d'origine biologique.

La production de biomasse obéit aux lois usuelles de la croissance des structures vivantes.

Dans le cas des plantes conservant leur intégrité, les méthodes conventionnelles de culture sont de préférence employées (en terre, en serre, hydroponie, etc).

Lorsque la structure vivante est une partie d'un végétal telle que cellule, tissu ou organe, la structure vivante peut donc avoir pour origine une plante dite supérieure, monocotylédone, comme les Lentilles d'eau ou dicotylédone et notamment parmi les Daucales, les Lianes, les Bignoniacées, les Morées, les Cornacées, les Cactacées.

Pour ce type de structure qui, par exemple, produit une matière tuffeuse et/ou travertineuse, il est possible d'utiliser le milieu de culture suivant:

| | |
|---|---|
| Eau distillée | 1 000,00 grammes |
| Nitrate de calcium | 0,71 grammes |
| Nitrate de potassium | 0,568 grammes |
| Sulfate de magnésium | 0,284 grammes |
| Phosphate d'ammonium | 0,142 grammes |
| Chlorure ferrique | 0,112 grammes |
| Iodure de potassium | 0,0028 grammes |
| Acide borique | 0,0005 grammes |
| Sulfate de Zinc | 0,0005 grammes |
| Sulfate de manganèse | 0,0005 grammes |

Par ailleurs, la structure d'origine végétale peut être une algue et notamment Rhodophyte, Chlorophyte, Cyanophyte, Chromophyte et/ou encore un Protophyte, et alors d'autres milieux sont envisageables.

Il en va de même si la structure vivante est un animal, ou partie d'animal. Les biomasses minérales peuvent être obtenues par culture in-vitro des cellules tissu et/ou organe impliqués dans les biosynthèses minérales du règne animal, mais aussi par recours à des productions actuelles et/ou fossiles.

Les micro-organismes sont également cultivables de façon abondante dans des fermenteurs industriels du commerce, et notamment des ultra-fermenteurs à filtrage tangentiel qui permettent d'obtenir une production en continu.

Une ou plusieurs structures vivantes compatibles peuvent être cultivées au sein d'un même milieu, éventuellement avec au moins une autre structure compatible d'origine végétale, animale et/ou micro-organique.

L'étape de préparation de la base peut comporter une phase de récolte ou collecte soit de la structure vivante précitée, soit du matériau minéral sédimentaire, et de son incorporation à la base dans des proportions telles que cet apport constitue au moins une partie du matériau minéral requis.

Il est possible de collecter le matériau minéral sous forme, par exemple, de dépôt, concrétionnement actuel et/ou fossile, et de l'incorporer à la base dans des proportions déterminées soit lors de l'étape de préparation, soit lors de l'étape de traitement exposée plus loin.

Par ailleurs, la structure vivante est choisie pour que sa matière minérale associée contienne au moins un constituant minéralogique carbonaté, siliceux, séléniteux ou analogues.

De nombreuses autres sources nouvelles de minéral sont utilisables conformément à l'invention. Le débroussaillage ou d'autres opérations agricoles (récoltes dont fenaison, moisson, vendanges et autres) engendrent d'importantes quantités de produits minéralisés, ou minéralisables, ou minéralisateurs, et aptes à une sur-minéralisation (foin, paille, chaume, etc.).

Certaines opérations industrielles engendrent des déchets d'origine végétale, telle la sciure, pouvant être minéralisés, et/ou surminéralisés, mais on peut aussi utiliser les déchets résultant de débroussaillage. Il en va de même pour les dépôts côtiers d'algues marines, source en partie du Maerl, des agrégats phyliteux (tels les micas) ou encore sphérules plastiques, etc.

L'étape de traitement vise à produire une base à minéralisation naturelle convenablement dosée, tant en éléments majeurs qu'en oligo-éléments, selon les déficits rencontrés lors des cultures.

Un autre avantage de l'invention est de pouvoir corriger une minéralisation naturelle déficiente dans un sol donné non seulement par incorporation d'une base extemporanée préalablement minéralisée et inactivée, mais aussi de permettre une biominéralisation in-situ - et active - des terreaux et/ou des sols par contact avec les micro-organismes et/ou organismes minéralisateurs choisis dans un milieu adéquat.

La seconde étape du procédé de l'invention consiste à traiter ladite base après sa surminéralisation de façon à la transformer en une matière minérale inactivée et texture prédéfinie en fonction de la nature et des besoins des sols agricoles.

En cas de production de biomasse, à partir d'une structure vivante, l'étape du traitement peut comprendre une phase d'inactivation de la structure vivante, consistant à interrompre tout développement cellulaire par adjonction d'un sel ou de substances, ayant des effets analogues sur la structure vivante retenue, dans des proportions convenables.
D'autres méthodes d'inactivation peuvent être employées seules ou en complément, dont l'irradiation et l'élévation à haute température. On peut recourir à la phase d'inactivation lors de l'élaboration du produit.

Une phase de malaxage peut être exécutée durant l'une des étapes du procédé et, de préférence, au moins en partie simultanément à une autre phase du procédé et notamment lors de l'inactivation.

Au moins l'un, parmi la biomasse, la base, le matériau (sous forme, par exemple, d'empilements minéraux lors de la culture de structures vivantes, ou encore sous forme de fossiles, déchets solides) et/ou la matière minérale, est fragmenté lors de l'étape de préparation et éventuellement lors de l'étape de traitement inactivant, pour leur emploi au sein de la base. La phase de fragmentation est effectuée jusqu'à obtention d'une texture ou malléabilité déterminée, et de préférence avec une granulométrie donnée. La fragmentation est au moins partiellement effectuée par dislocation à l'aide d'ultra-sons et/ou de moyens physico-chimiques tels qu'adjonction d'additifs, traitement cryogénique et/ou thermique, broyage ou variation de pression. Dans ce cas, l'une des phases du procédé peut consister à élaborer une suspension de la biomasse ou du matériau fragmenté dans un liquide, de préférence, aqueux.

Il est possible d'incorporer lors d'une phase du procédé, au moins à l'un parmi le milieu de culture, la biomasse, le matériau, la base et la matière minérale, une substance colorante de nuance prédéterminée. Cette substance est éventuellement un élément de la biomasse.

Une étape de préparation et/ou de traitement peut consister à incorporer à l'un au moins parmi la biomasse, le milieu, le matériau, la base et la matière minérale, un agent de cohésion ou texture, de proportions et composition telles que la matière finale présente une structure, une dureté ou une élasticité prédéterminée. Cet agent est, de préférence, un liant, notamment métallique tel que calcium, magnésium, silicium, baryum, sodium, fluor, aluminium, fer, manganèse, zinc, ou organique tel que collagène, muco-polysaccharide, et composé poly-cellulosique. Selon la composition de cet agent, celui-ci peut faire office de composé d'inactivation dans le cas d'une base comportant une structure vivante cultivée ou collectée.

Durant l'une des phases ou étapes du procédé, un additif, par exemple moussant, fibreux, agglomérant, peut être incorporé à la base et/ou à la matière minérale précitée.

La substance nutritive finale peut être, suivant les étapes suivies et les additifs incorporés à la base, un solide, un aérosol, un fluide, ...

Un matériau (inerte) peut également être mélangé à la substance nutritive pour former notamment un terreau.

Par ailleurs, le procédé peut comprendre une phase de déshydratation au moins partielle d'un ou plusieurs composants de la base et/ou de la matière minérale précitée. Cette phase de déshydratation peut être au moins partiellement effectuée par filtrage, irradiation, centrifugation, traitement thermique et/ou cryogénique.

Il est envisageable, suivant le procédé, que les phases d'inactivation, déshydratation et fragmentation, soient au moins partiellement effectuées simultanément.

L'étape de traitement peut comprendre une phase de conditionnement dans un emballage approprié de la matière minérale qui constitue un produit fini.

Selon un exemple de déroulement des phases du procédé, la base est constituée par une biomasse, obtenue par culture in vitro d'un micro-organisme minéralisateur, en présence de cellules végétales résiduelles issues du broyat de broussailles, copeaux, sciure, etc. La suspension biominérale obtenue est colorée puis séchée et le mélange est alors malaxé et subit un apport de sel d'inactivation. Cette poudre de composition adaptée à la demande est utilisable comme substance nutritive telle qu'engrais ou amendements divers, après séchage.

Le procédé décrit dans la présente demande, selon l'invention concerne également un procédé de production biologique de substances naturelles biominérales d'origine animale et/ou végétale et destinées à des usages thérapeutiques médicaux et/ou paramédicaux ainsi que les substances obtenues par ce procédé.

On connaît des procédés de production de drogues végétales consistant à cueillir et/ou récolter des plantes naturelles et/ou à cultiver des espèces largement commercialisées. Cependant, les produits ainsi obtenus sont soumis aux aléas de la nature comme toute activité agricole et/ou horticole de la production à la conservation et préparation du produit. De surcroît, le stock des productions soumises à de telles variabilités doit presque toujours être renouvelé chaque année.

Par ailleurs, on connaît depuis longtemps l'existence de nombreux composés de nature minéralogique variée et souvent complexe (calcique ou silicique par exemple), visibles à l'état figuré dans les cellules ou d'origine cellulaire. Il s'agit de sels ou de composés chimiquement complexes souvent considérés comme des déchets de la vie cellulaire que l'organisme ne réemploie pas. La présence de ces substances est généralement expliquée par le fait que l'organisme les absorbe en quantités supérieures à ses besoins et qu'il se débarrasse du surplus sous forme cristallisée lors ce réactions Durement physicochimiques. Parmi les exemples les plus connus de substances produites par des végétaux, on peut citer :
- la gentianistérine qui est une phytostérine présente sous forme d'aiguilles, contenue dans les cellules de la racine des Gentianes (famille des Gentianacées);
- le calcium, potassium, magnésium, silice, fer et chlore contenus dans la Garance (famille des Rubiacées);
- la navelrine extraite des feuilles de pervenche de Madagascar et modifiée par hémi-synthèse;
- le taxotère à base de feuilles d'If...

Les principes extraits des plantes médicinales et/ou aromatiques ne représentent pas d'une manière exacte les propriétés médicamenteuses de la drogue: par exemple les solanées mydriatiques, l'opium, le quinquina, la coca, le kola, la digitale, les strophantus, dérivés anthraquinoniques, etc. possèdent comme on le sait une action globale différente de l'atropine, de la morphine, de la quinine, de la cocaïne, de la caféine, des digitalines ou strophantines, de l'emodine, etc. Il est souvent impossible d'extraire des matières premières végétales, des sucs voire même des produits complexes définis avec plus de précision, représentatifs de leur principe actif.

La présente invention remédie à ces inconvénients et propose un procédé de production biologique de substances minérales à usage médical et/ou para-médical, indépendantes des changements de l'environnement naturel extérieur, permettant des dosages médicaux et/ou paramédicaux précis, permettant chacune ou en association de subvenir à des carences nutritionnelles et/ou pathologiques et permettant encore des traitements médicaux notamment phytothérapiques.

Le procédé est caractérisé par les étapes consistant à:
- préparer une base comprenant une quantité prédéterminée d'un matériau minéral, synthétisé dans la nature ou industriellement par au moins une ou partie de structure vivante appartenant au règne végétal et/ou animal.
- traiter éventuellement ladite base de façon à la transformer en une matière minérale inactivée et de texture prédéfinie en fonction d'un usage médical et/ou para-médical déterminé.

L'invention prévoit l'usage de toute 〈〈structure vivante〉〉 à pouvoir pétrifiant. Par "structure vivante" minéralisatrice, minéralisée ou minéralisable, il faut comprendre toute structure cellulaire ou d'origine cellulaire (végétale, animale ou micro-organique), vivante et/ou résultant de la vie et/ou des composés d'origine biologique, cristallisés ou non, tels des enzymes, hormones, protéines, ADN, etc.

Par "matière inactivée", il faut comprendre matière conforme à l'invention et dénuée de toute activité biologique et/ou biominéralisatrice notamment de toute activité microbiologique pathogène.

Le terme 〈〈 minéral 〉〉 doit être compris au sens large, à savoir comme incluant dans sa composition un minéral.

Mais d'autres avantages et particularités de l'invention ressortiront mieux de la description détaillée d'exemples de sa mise en pratique et qui sont expliqués ci-après.

Le procédé de fabrication ou obtention d'une matière ou composition minérale inactivée à usage extemporané comprend principalement deux étapes.

La première consiste à préparer une base comprenant une quantité prédéterminée d'un matériau minéral synthétisé par au moins une ou partie de structure vivante sélectionnée parmi le règne végétal, animal et/ou les micro-organismes.

La classification ci-après donne des exemples non limitatifs de structures vivantes aptes à être sélectionnées pour l'application de l'invention.

Dans le règne végétal, on peut avoir recours tout aussi bien à des végétaux supérieurs dicotylédones, monocotylédones qu'à des végétaux inférieurs thallophytiques ou microorganiques.
a) - Parmi les Dicotylédones:
   - les cellules de l'ordre des Daucales, famille des Araliacées, genre Hedera dont Hedera helix;
   - les cellules de l'ordre des Arales, genre Rhaphidophora, espèce Syngonium podophyllum (Schott) "albolineatum", espèces Syngonium auritum syn. Philodendron trifolium et espèce Syngonium hastifolium;
   - les cellules de l'ordre des Solanales, famille des bignoniacées, espèce Catalpa bignonioidées.

   De surcroît, toute cellule végétale, même celle qui serait peu symbiotique est susceptible par le procédé de l'invention d'être minéralisée et/ou surminéralisée si on la met en contact avec de tels micro-organismes minéralisateurs. C'est le cas des cellules précitées, mais aussi de toutes cellules végétales issues par exemple de broussailles sous forme de copeaux, de sciures et qui par ce procédé se pétrifient, c'est-à-dire se transforment en substrat pétrifié, carbonaté et/ou silico-carbonaté.
b) - Parmi les Monocotylédones:
   - les cellules de l'ordre des Pandarales dont celles de la famille des Typhacées;
   - les cellules de graminées dont celles des Lemnacées.
c) - Parmi les fougères:
   Les épidermes chez Equisetum arvense (Prêle commune)sont le théâtre de productions minérales de silice souvent considérées comme des concrétions, issues de sécrétions membranaires épidermiques alors que s'y trouvent impliquées des bactéries en périphérie et/ou dans les cellules d'Equisetum arvense. Celles-ci expliquent les dépôts de silice intra et/ou extra-cellulaires.
d) - Parmi les algues:
   Des masses biominérales résultant de la multiplication et de l'accumulation du frustules d'algues de façon naturelle ou industrielle sont applicables à l'invention. Par exemple:
   - les Rhodophytes avec principalement les Némalionales, les Solénoporacées et les Corallinacées (comportant les Lithothamnium, les Mélobésiées);
   - les Chromophytes qui réunissent notamment les Chromophycées et surtout les Diatomophycées aux productions siliceuses.
   - les Chlorophytes avec les Siphonales (comportant les Codiacées dont Halimeda), les Dacycladales (comportant les dacycladacées dont Acétabularia);
   - les Cyanophytes avec les Porostromata et les Spongiostroma dont Rivularia Oscillatoria Phormidium Chroococcus et Gléocapsa sont aptes a être selectionnés.
e) - Parmi les Lichens:
   La masse biominérale peut aussi résulter de la culture d'une association végétale tripartite de type lichénique. Les Lichens sont des associations végétales composites. L'association dite à bénéfice réciproque se compose d'un champignon, d'une algue et de bactéries minéralisantes, dites bactéries symbiotes, semblables à celles visées précédemment. Ces bactéries contribuent à des incrustations minérales complexes et pigmentées au sein du mycélium.
f) - Parmi les champignons et leurs spores;
g) - Parmi les micro-organismes:
   virus ou bactéries donc bacillus mégathérium, pseudomonas fluorescens,pseudomonas maltophilia, pseudomonas putida, buttauxiella agrestis rhodococcus, serratia marescens.

Le procédé est utilisable avec des micro-organismes aptes à fixer du calcium, calcium magnésien, magnésium, phosphore, aluminium, manganèse, fer, silice ou d'autres substances intervenant à doses infinitésimales. Ces micro-organismes sont largement répandus dans la nature.

La structure vivante peut aussi être un animal ou une partie d'animal telle que cellule, tissu et/ou organe choisi parmi les protozoaires ou les métazoaires invertébrés tels les spongiaires, les lamellibranches et les échinodermes ou encore parmi les vertébrés.

De préférence, l'étape de préparation de la base comporte une phase consistant à cultiver la structure vivante précitée durant la période et dans un milieu tels qu'au moins une partie dite "biomasse minérale" dudit matériau est alors produite ou synthétisée par cette structure.

A titre d'exemple, l'une ou plusieurs des structures vivantes correspondant aux minéraux et/ou aux roches ci-après peuvent être sélectionnées conformément à l'invention:
Cellule de Hedera helix <---> Oxalate de calcium / Calcaire
Cellule de Ficus élastica <--> Carbonate de calcium / Calcaire
Cellule d'Equisetum <--> Silice / Opaline / Bois silicifié / Grés
Cellule de Graminées <--> Silice / Opaline / Bois silicifié / Grès
Cellule de Typhacées <--> Silice / Opaline / Bois silicifié / Grés
Champignon Ascomycète <--> Dipicolinate de calcium / Calcaire
Cellule de Pectascinacées <--> Carbonate de calcium / Calcaire
Algue Chromophyte <--> Silice / Diatomite
Algue Rhodophyte <--> Carbonate de calcium / Calcaire / Travertin
Algue Chlorophyte <--> Carbonate de calcium / Calcaire / Travertin
Algue Cyanophyte <--> Carbonate de calcium / Calcaire / Travertin
Mollusque Lamellibranche <--> Carbonate de calcium / Calcaire / Grès coquilliers

La première étape de préparation a pour but d'obtenir une base minérale c'est-à-dire un produit intermédiaire. De préférence, l'étape de préparation de la base comporte une phase consistant à cultiver la structure vivante précitée durant une période et dans un milieu tels qu'au moins une partie dite 〈〈 biomasse minérale 〉〉 du dit matériau est alors produite ou synthétisée par cette structure en utilisant par exemple le milieu de culture suivant:

| | |
|---|---|
| - eau distillée | 1 000 g |
| - nitrate de calcium | 0,71 g |
| - nitrate de potassium | 0,568 g |
| - sulfate de magnésium | 0,284 g |
| - phosphate d'ammonium | 0,142 g |
| - chlorure ferrique | 0,112 g |
| - iodure de potassium | 0,0028 g |
| - acide borique | 0,0005 g |
| - sulfate de zinc | 0,0005 g |
| - sulfate de manganèse | 0,0005 g |
| mais d'autres milieux sont envisageables. | |

Il en va de même si la structure vivante est un animal ou partie d'animal. Les biomasses minérales sont susceptibles d'être obtenues par culture in vitro des cellules, tissus et/ou organes impliqués dans les biosynthèses minérales du règne animal mais aussi par recours à des productions naturelles.

Selon les cas, la structure sélectionnée est cultivée notamment in vivo, en terre, sur couche riche en matière organique, par hydroponie, en boite de Pétri, par élevage en batterie, sur pieds ou pisciculture, ou dans un. réacteur tel que fermenteur. Ainsi, les microorganismes sont cultivables de façon abondante dans des fermenteurs industriels du commerce, et notamment des ultrafermenteurs à filtrage tangentiel, qui permettent d'obtenir une production en continu.

La structure est également susceptible d'être cultivée dans un milieu nutritif approprié connu de l'homme de l'art tel que liquide de Knopp, solution d'Earle, Hanks, milieu dit "199", milieu de Sabouraud, milieu MEM-Eagle ou analogues.

Une ou plusieurs structures vivantes compatibles peuvent être cultivées ou sein d'un même milieu, éventuellement avec au moins une autre structure compatible d'origine végétale, animale, et/ou micro-organique.

De manière avantageuse, les sels synthétisés dans l'organisme, décrits plus haut comme les déchets de la vie cellulaire que l'organisme ne remploie pas, ne résultent pas de simples réactions physico-chimiques et des micro-organismes minéralisateurs sont fréquemment impliqués dans ces dépôts de cellules végétales. Ces micro-organismes minéralisateurs peuvent être multipliés à l'intérieur de ces cellules ou en être extraits et multipliés in vitro en mono ou pluricouches constituant alors de véritables tissus, ou sous forme d'agrégats polymorphes.

La seconde étape consiste à traiter ladite base après sa surminéralisation de façon à la transformer en une matière minérale inactivée de texture prédéfinie en fonction de l'usage thérapeutique.

En cas de production de biomasse à partir d'une structure vivante, l'étape du traitement peut comprendre une phase d'inactivation de la structure vivante. Par inactivation, il faut comprendre obtention de matière inactivée à partir de la structure vivante ("matière inactivée" et "structure vivante") ayant été définis auparavant. Cette phase d'inactivation peut être effectuée par adjonction d'un sel.

La biomasse est stabilisée, c'est-à-dire inactivée de façon que tout développement cellulaire soit interrompu. Un tel résultat peut être obtenu par ajout dans des proportions convenables de substances ayant des effets analogues sur la structure vivante retenue.

D'autres méthodes d'inactivation peuvent être employées, seules ou en complément, dont l'irradiation et l'élévation à haute température (température supérieure à celle que la structure vivante apporte). Une phase de malaxage peut être exécutée au moins en partie simultanément à une autre phase du procédé et notamment lors de l'inactivation.

On peut recourir à la phase d'inactivation lors de l'élaboration du produit.

L'étape de préparation de la base peut comporter une phase consistant à récolter ou collecter la structure vivante et l'incorporer à la base dans des proportions telles que cet apport constitue au moins une partie du produit minéral final. Le matériau minéral ainsi récolté et/ou collecté peut être de nature variée : carbonatée, siliceuse, saline, fluorée, barytée, carbonée, ferromanganique, sous forme de dépôt, concrétionnement actuel et/ou fossile et peut être incorporé à la base soit lors de l'étape de préparation, soit lors de l'étape de traitement.

Par ailleurs, la structure vivante est choisie pour que se substance biominérale associée contienne au moins l'un des constituants suivants : précipités calciques, siliciques, fluorés, ferrugineux et autres ou contienne au moins un constituant minéralogique carbonaté, siliceux, séléniteux ou analogues.

De nombreuses autres sources nouvelles de biominéralisation sont utilisables conformément à l'invention.

Durant une phase possible du procédé, au moins l'une parmi la biomasse, la base, la substance biominérale précitée est fragmentée lors de l'étape de préparation, et éventuellement lors de l'étape de traitement inactivant. Par exemple, si lors de la culture d'une structure vivante, on obtient des empilements minéraux et/ou stratifiés, qu'on peut éventuellement morceler pour leur emploi au sein de la base. De même, si une partie ou la totalité de la substance biominérale résulte d'une collecte, elle peut être broyée ou fragmentée.

La phase de fragmentation est effectuée jusqu'à obtention d'une texture ou malléabilité déterminée: la fragmentation a pour but d'obtenir une substance de préférence avec une granulométrie donnée. La fragmentation est au moins partiellement effectuée par dislocation à l'aide d'ultrasons et/ou de moyens physicochimiques, tels qu'adjonction d'additifs, traitement cryogénique et/ou thermique, broyage ou variation de pression.

Dans ce cas, l'une des phases du procédé consiste à élaborer une suspenson de la biomasse ou du matériau fragmenté précité dans un liquide, de préférence aqueux, pour pulvérisation ou application par un tout autre moyen. Ce liquide peut entrer dans la composition de l'un des milieux de culture précités.

Il est possible d'incorporer lors d'une phase du procédé au moins l'un parmi le milieu de culture, la biomasse, le matériau, la base et la matière minérale, une substance colorante de nuance prédéterminée. Cette substance est éventuellement un élément de la biomasse.

Une étape de préparation et/ou de traitement peut consister à incorporer à l'un au moins parmi la biomasse, le milieu, le matériau, la base et la matière minérale un agent de cohésion ou texture. Cet agent est de préférence un liant, notamment, métallique tel que calcium, magnésium, silicium, baryum, sodium, fluor, aluminium, fer, manganèse, zinc ou organique tel que collagène, muco-polysaccharide et composé polycellulosique. Cet agent fait éventuellement office de composé d'inactivation dans le cas d'une base comportant une structure vivante cultivée ou collectée. Avantageusement, les proportions et la composition de l'agent de cohésion et/ou de texture précité sont choisies pour que la matière finale présente une spécificité prédéterminée.

Durant l'une des phases ou étapes du procédé, un additif, par exemple moussant, effervescent, fibreux, supplément aromatique, odorant, ou analogue, peut être incorporé à la base et/ou à la matière minérale précitées.

Par ailleurs, le procédé est caractérisé en ce qu'il comprend une phase de déshydratation au moins partielle d'un ou plusieurs composants de la base et/ou de la matière minérale précitées. Cette phase de déshydratation peut être au moins partiellement effectuée par filtrage, irradiation, centrifugation, traitement thermique et/ou cryogénique.

Il est envisageable, suivant le procédé que les phases d'inactivation, déshydratation et fragmentation soient au moins partiellement effectuées simultanément.

L'étape de traitement peut comprendre une phase de conditionnement dans un emballage approprié de la matière minérale qui constitue un produit fini.

Un exemple de déroulement des phases du procédé peut être envisagé conne suit : la base est constituée par une biomasse obtenue par culture in vitro d'un micro-organisme minéralisateur en présence de cellules végétales issues d'une plante médicinale. La suspension biominérale obtenue est séchée, et le mélange est alors malaxé et subit un apport de sels d'inactivation, l'ensemble constituant une poudre de composition adaptée à la demande.

Le procédé décrit dans la présente demande, selon l'invention concerne également un procédé de production biologique de substrats minéraux, notamment de parois, membranes, films, tissus et fils, biominéraux, d'origine naturelle et/ou artificielle, ainsi que le substrat obtenu par ce procédé.

On connaît, dans le domaine par exemple des systèmes phoniques, acoustiques ou électroacoustiques, notamment les haut-parleurs, des membranes en carton ou des membranes métalliques. Cependant, les membranes en carton restituent incorrectement les sons transitoires et les membranes métalliques ne résonnent qu'à certaines fréquences.

Inversement, les isolants phoniques dans le domaine du bâtiment n'amortissent pas tous les sons et n'insonorisent donc pas de manière convenable.

Dans le domaine du textile, de nombreux types de tissus sont d'origine artificielle et leurs caractéristiques, notamment en souplesse, sont prédéterminées et inchangeables.

La présente invention remédie à ces inconvénients et propose un procédé de production d'un substrat notamment paroi, membrane, film, tissu et fil, d'origine naturelle et/ou artificielle,
caractérisé par les étapes consistant à:
- préparer une base comprenant une quantité prédéterminée d'un matériau biominéral, synthétisé par au moins une ou partie de structure vivante choisie parmi le règne végétal, animal et/ou les micro-organismes ; et
- traiter ladite base de façon à la transformer en un substrat biominéral inactivé de texture prédéfinie en fonction d'un usage extemporané choisi, notamment comme isolant thermique, phonique, filtre, membrane acoustique, paroi décoratrice, tissu, fil, film ;
   afin d'améliorer à la fois la structure, la composition biochimique et/ou biologique et/ou les caractéristiques physico-chimiques des substrats naturels et/ou artificiels utilisés notamment dans les industries du textile et du bâtiment, dans les domaines de l'agriculture, l'horticulture, l'agronomie et de la protection de l'environnement, notamment par filtration, dans les industries des systèmes phoniques, acoustiques ou électroacoustiques.

Dans le domaine agricole, ces nouveaux substrats pourront être protecteurs (contre l'érosion, le déssèchement, etc;...) et/ou nutritifs vis-à-vis des sols et/ou des cultures.

Dans le domaine de la filtration, ils agiront comme filtres biominéraux et biomasses minérales en vue de l'amélioration des conditions absorbantes desdites biomasses pour décontaminer les eaux usées, notamment détoxiquer des eaux riches en métaux afin de les récupérer.

Avantageusement, on réalise la biominéralisation in situ dans les substrats organiques et/ou inorganiques par contact avec des micro-organismes et/ou des organismes minéralisateurs choisis dans un environnement adéquat.

Suivant une caractéristique de l'invention, l'étape de préparation de la base comporte une phase consistant à cultiver la structure vivante durant une période et dans un milieu tels qu'au moins une partie dite "biomasse" dudit matériau est alors produite ou synthétisée par cette structure.

Avantageusement, la biomasse est obtenue par culture in vitro sur supports nutritifs variés, notamment solides, rigides, semi-rigides, souples ou encore liquides.

Suivant une autre caractéristique de l'invention, l'activité biominéralisatrice de micro-organismes dans l'étape de traitement tels que les champignons, notamment un mycélium d'ascomycète, du genre tuber, les bactéries notamment pseudomonas fluorescens ou pseudo putida ou bacillus species ou buttauxiella agrestis ou serratia marcescens ou rhodococcus, les algues sur des supports nutritifs rigides, semi-rigides ou souples, élastiques, aboutit à la constitution de membranes biominérales.

De manière avantageuse, lesdites membranes biominérales sont constituées d'une trame organique minéralisée où la minéralisation biogène microscopique et/ou macroscopique est automorphe et/ou xénomorphe et/ou amorphe, permettant la transmission des vibrations, y compris des sons aigus de façon rapide, bien équilibrée et dépourvue de résonance interne.

De manière avantageuse également, l'invention permet, en choisissant comme micro-organisme une algue brune telle diatomée - seule ou en symbiose avec un champignon, un mycélium et/ou plusieurs bactéries - de réaliser un biofilm plus ou moins souple présentant les caractéristiques spécifiques aux biofilms (les biofilms diatomitiques naturels actuels en sont un exemple).

La présente invention concerne également un substrat biominéralisé, susceptible d'être inactivé, à usage extemporané choisi, obtenu par le précédé précédent. Ce substrat présente l'avantage d'apporter une porosité favorisant les échanges gazeux et/ou les phénomènes d'absorption et d'adsorption comme c'est le cas avec par exemple les diatomites fossiles.

L'invention prévoit l'usage de toute "structure vivante" à pouvoir pétrifiant. Par "structure vivante" minéralisatrice, minéralisée ou minéralisable, il faut comprendre toute structure cellulaire ou d'origine cellulaire (végétale, animale ou micro-organique), vivante et/ou résultant de la vie et/ou des composés d'origine biologique, cristallisés ou non, tels des enzymes, hormones, protéines, ADN, ...

Par "matière inactivée", il faut comprendre matière conforme à l'invention et dénuée de toute activité biologique et/ou biominéralisatrice, notamment de toute activité micro-biologique pathogène.

Les sources de matériau minéral disponible pour la mise en oeuvre du procédé sont considérables d'autant qu'il est possible d'employer des structures vivantes provenant de récupération ou recyclage de déchets ou sous-produits constituant un apport biominéral ou apte à générer un apport biominéral, en les valorisant.

Le terme "biominéral" doit être compris ici au sens large, à savoir 'comme incluant dans sa composition un minéral.

D'autres avantages et particularités de l'invention ressortiront mieux de la description détaillée d'exemples de sa mise en pratique, et qui sont expliqués ci-après.

Le procédé de fabrication ou obtention d'un substrat minéral inactivé à usage extemporané comprend principalement deux étapes.

La première consiste à préparer une base comprenant une quantité prédéterminée d'un matériau biominéral synthétisé par au moins une ou partie de structure vivante, sélectionnée parmi le règne végétal, animal et/ou les micro-organismes. Cette base biominérale constitue un produit intermédiaire.

De préférence, l'étape de préparation de la base comporte une phase consistant à cultiver la structure vivante précitée durant une période et dans un milieu tels qu'au moins une partie dite "biomasse minérale" dudit matériau est alors produite ou synthétisée par cette structure.

La classification ci-après donne des exemples non limitatifs de structures vivantes aptes à être sélectionnées pour l'application de l'invention:
a) - Parmi les Dicotylédones:
   - Les cellules de l'ordre des Daucales, famille des Araliacées, genre Hedera dont Hedera helix ;
   - Les cellules de l'ordre des Arales, genre Rhaphidophora, espèce Syngonium podophyllum (Schott), "Albolineatum", espèces Syngonium auritum syn. Philodendron trifolium et espèce Syngonium hastifolium;
   - Les cellules de l'ordre des Solanales, famille des Bignoniacées, espèce Catalpa bignonioidées.

   De surcroît, toute cellule végétale, même celle qui serait peu symbiotique est susceptible, par le procédé de l'invention, d'être minéralisée et/ou surminéralisée si on la met en contact avec de tels micro-organismes minéralisateurs.
   C'est ainsi le cas des cellules précitées, mais aussi de toutes les cellules végétales issues par exemple de broussailles sous forme de copeaux, de sciure, et qui par ce procédé se pétrifient c'est-à-dire se transforment en substrat pétrifié, carbonaté et/ou silico-carbonaté.
b) - Parmi les Monocotylédones :
   - Les cellules de l'ordre des Pandarales dont celles de la famille des Typhacées ;
   - Les cellules de graminées dont celles des Lemnacées.
c)- Parmi les Fougères :
   Les épidermes chez Equisetum arvense (Prêle commune) sont le théâtre de productions minérales de silice souvent considérés comme des concrétions issues, de sécrétions membranaires épidermiques alors que s'y trouvent impliquées des bactéries en périphérie et/ou dans les cellules d'Equisetum arvense. Celles-ci expliquent les dépôts de silice intra et/ou extracellulaire.
d) - Parmi les Algues:
   Des masses biominérales résultant de la multiplication et de l'accumulation de frustules d'algues de façon naturelle ou industrielle sont applicables à l'invention. Par exemple :
   - Les Rhodophytes avec principalement les Némalionales, les Solénoporacées et les Corallinacées (comportant les Lithothamnium, les Mélobésiées);
   - Les Chlorophytes avec les Siphonales (comportant les Codiacées dont Halimeda) et les Dacycladales (comportant les dacycladacées dont Acétabularia);
   - Les Chromophytes qui réunissent notamment les Chromophycées et surtout les Diatomophycées aux productions siliceuses;
   - Les Cyanophytes avec les Porostromata et les Spongiostroma dont Rivularia, Oscillatoria, Phormidium, Chroococcus et Gleocapsa sont aptes à être sélectionnés.
e) - Parmi les Lichens :
   La masse biominérale peut aussi résulter de la culture d'une association végétale tripartite de type lichénique. Les Lichens sont des associations végétales composites. L'association dite à bénéfice réciproque se compose d'un champignon, d'une algue et de bactéries minéralisantes, dites bactéries symbiotes, semblables à celles visées précédemment. Ces bactéries contribuent à des incrustations minérales complexes et pigmentées au sein du mycélium.
f) - Parmi les Champignons et leurs spores.
g) - Parmi les micro-organismes : virus ou bactérie dont bacillus mégathérium, pseudomonas fluorescens, pseudomonas maltophilia, pseudomonas putida, buttauxiella agrestis, rhodococcus, serratia marescens.

Le procédé est utilisable avec des micro-organismes aptes à fixer du calcium, magnésium, phosphore, aluminium, manganèse, fer, silice ou d'autres substances intervenant à doses infinitésimales. Ces micro-organismes sont largement répandus dans la nature.

La structure vivante peut aussi être un animal, ou partie d'animal telle que cellule, tissu et/ou organe, choisi parmi les protozoaires ou les métazoaires invertébrés tels les spongiaires, les lamellibranches et les échinodermes ou encore parmi les vertébrés.

De préférence, l'étape de préparation de la base comporte une phase consistant à cultiver la structure vivante précitée durant une période et dans un milieu tels qu'au moins une partie dite "biomasse minérale" dudit matériau est alors produite ou synthétisée par cette structure.

A titre d'exemple, l'une ou plusieurs des correspondances entre les structures vivantes et les minéraux et/ou les roches ci-après, peuvent être sélectionnés conformément à l'invention:
Cellules de Hedera helix ↔ Oxalate de calcium/ Calcaire
Cellules de Ficus élastica ↔ Carbonate de calcium/ Calcaire
Cellules d'Equisetum <-->Silice / Opaline / Bois silicifié /Grès
Cellules de Graminées <-->Silice / Opaline / Bois silicifié /Grès
Cellules de Typhacées <-->Silice / Opaline / Bois silicifié /Grès
Champignon Ascomycète <--> Dipicolinate de calcium/ Calcaire
Cellules de Pectascinacées ↔ Carbonate de calcium/ Calcaire
Algue Phéophysée↔Silice / Diatomite /
Algue Rhodophycée,↔Carbonate de calcium / Calcaire / Travertin
Algue Chlorophycée,↔Carbonate de calcium / Calcaire / Travertin
Algue Cyanophycée ↔Carbonate de calcium / Calcaire / Travertin
Mollusque Lamellibranche<-->Carbonate de calcium / Calcaire /Grès coquilliers

Le procédé de l'invention permet ainsi de produire des substrats carbonatés ou siliceux, ou silico-carbonatés par biominéralisation, ces substrats n'étant pas obligatoirement d'origine biologique.

La production de biomasse obéit aux lois usuelles de la croissance des structures vivantes.

Dans le cas des plantes conservant leur intégrité, les méthodes conventionnelles de culture sont de préférence employées (en terre, en serre, hydroponie, etc).

Lorsque la structure vivante est une partie d'un végétal telle que cellule, tissu ou organe, la structure vivante peut donc avoir pour origine une plante dite supérieure, monocotylédone, comme les Lentilles d'eau ou dicotylédone et notamment parmi les Daucales, les Lianes, les Bignoniacées, les Morées, les Cornacées, les Cactacées.

Pour ce type de structure qui, par exemple, produit une matière tuffeuse et/ou travertineuse, il est possible d'utiliser le milieu de culture suivant:

| | |
|---|---|
| Eau distillée | 1 000,00 grammes |
| Nitrate de calcium | 0,71 grammes |
| Nitrate de potassium | 0,568 grammes |
| Sulfate de magnésium | 0,284 grammes |
| Phosphate d'ammonium | 0,142 grammes |
| Chlorure ferrique | 0,112 grammes |
| Iodure de potassium | 0,0028 grammes |
| Acide borique | 0,0005 grammes |
| Sulfate de Zinc | 0,0005 grammes |
| Sulfate de manganèse | 0,0005 grammes |

Par ailleurs, la structure d'origine végétale peut être une algue et notamment Rhodophyte, Chlorophyte, Schyzophyte, Cyanophyte, Chromophyte et/ou encore un Protophyte, et alors d'autres milieux sont envisageables.

Il en va de même si la structure vivante est un animal, ou partie d'animal. Les biomasses minérales peuvent être obtenues par culture in-vitro des cellules tissu et/ou organe impliqués dans les biosynthèses minérales du règne animal, mais aussi par recours à des productions actuelles et/ou fossiles.

Les micro-organismes sont également cultivables de façon abondante dans des fermenteurs industriels du commerce, et notamment des ultra-fermenteurs à filtrage tangentiel qui permettent d'obtenir une production en continu.

Une ou plusieurs structures vivantes compatibles peuvent être cultivées au sein d'un même milieu, éventuellement avec au moins une autre structure compatible d'origine végétale, animale et/ou micro-organique.

La biominéralisation est donc réalisée en incorporant à la base, dans des proportions déterminées, des structures organiques et/ou inorganiques préalablement minéralisées.

L'invention peut également être mise en oeuvre non plus en incorporant à la base des structures organiques et/ou inorganiques minéralisées, mais in situ dans les parois, membranes, films, tissus et fils organiques et/ou inorganiques, par contact avec des micro-organismes et/ou organismes minéralisateurs choisis dans un milieu adéquat.

En effet, on a signalé depuis fort longtemps, l'existence de nombreux composés de nature minéralogique variée et souvent complexe (calcique ou silicique par exemple), visibles à l'état figuré, dans les cellules ou structures d'origine cellulaire. Il s'agit de sels ou de composés chimiquement complexes synthétisés dans l'organisme, résultant soit de simples réactions physico-chimiques, soit de l'activité de micro-organismes minéralisateurs.

Ces micro-organismes minéralisateurs peuvent être multipliés à l'intérieur de ces cellules ou en être extraits et multipliés in vitro dans divers substrats organiques et/ou inorganiques, constituant alors des structures biominérales d'accueil pouvant être tissées, découpées, collées, ... Ce phénomène, base du procédé de l'invention, est source de précipités non seulement calciques et/ou siliciques mais aussi magnésien, ferromanganiques, fluorés, barytés ou autres. Il s'observe notamment dans tout le règne végétal, ausi bien chez les végétaux supérieurs que chez les végétaux inférieurs. Dans la nature, d'une façon générale, ces dépôts peuvent contribuer à édifier les membranes et/ou les parois et/ou intervenir dans la physiologie cellulaire. On considère généralement que ces imprégnations (calciques et/ou siliciques et/ou autres) sont uniquement sous la dépendance de la physiologie globale de l'organisme, ou d'organe(s) particulier(s). Or, les micro-organismes minéralisateurs, présents (calcifiants et/ou silicifiants et/ou autres) participent, par des échanges symbiotiques ou d'autre nature, à la minéralisation. Le concept sur lequel repose le procédé de la présente invention réside en la présence et la participation effective, quasigénérale, des micro-organismes à pouvoir minéralisateur et entraîne la possibilité de la présente application.

La seconde étape consiste à traiter ladite base après sa surminéralisation, de façon à la transformer en une matière biominérale inactivée et de texture prédéfinie en fonction de notre usage présent.

En cas de production de biomasse à partir d'une structure vivante, l'étape de traitement peut comprendre une phase d'inactivation de la structure vivante. Par inactivation, il faut comprendre obtention de matière inactivée telle que définie précédemment à partir de structures vivantes telles que définies précédemment.

Cette phase d'inactivation peut être effectuée par adjonction d'un sel. La biomasse est stabilisée, c'est-à-dire inactivée de façon que tout développement cellulaire soit interrompu. Un tel résultat peut être obtenu par ajout dans des proportions convenables de substances ayant des effets analogues sur la structure vivante retenue. D'autres méthodes d'inactivation peuvent être employées, seules ou en complément, dont l'irradiation et l'élévation à haute température.

Une phase de malaxage/compactage/filature/tissage peut être exécutée durant l'une des étapes du procédé et, de préférence, postérieurement à l'inactivation.

On peut recourir à la phase d'inactivation lors de l'élaboration du produit.

L'étape de préparation de la base peut comporter une phase consistant à récolter ou collecter soit la structure vivante précitée soit le matériau biominéral et à l'incorporer à la base dans des proportions telles que cet apport constitue au moins une partie du matériau biominéral requis. Il est possible de collecter le matériau biominéral sous forme par exemple de dépôt, concrétionnement actuel et/ou fossile, et de l'incorporer à la base soit lors de l'étape de préparation, soit lors de l'étape de traitement.

Par ailleurs, la structure vivante est choisie pour que sa matière minérale associée contienne au moins un constituant minéralogique carbonaté, siliceux, séléniteux ou analogues.

De nombreuses autres sources biominérales sont utilisables conformément à l'invention. Le débroussaillage ou d'autres opérations agricoles (récoltes dont fenaisons, moissons, vendanges et autres) génèrent d'importantes quantités de produits minéralisés ou minéralisables ou minéralisateurs et aptes à une surminéralisation (foin, caille, chaume etc...).

La phase de culture est effectuée jusqu'à obtention d'une texture ou malléabilité déterminée et l'une des phases du procédé peut consister à élaborer une suspension de la biomasse ou du matériau fragmenté dans un liquide, de préférence aqueux.

Il est possible d'incorporer lors d'une phase du procédé, au moins à l'un parmi le milieu de culture, la biomasse, le matériau, la base et la matière minérale, une substance colorante de nuance prédéterminée. Cette substance est éventuellement un élément de la biomasse.

Une étape de préparation et/ou de traitement peut consister à incorporer à l'un au moins parmi la biomasse, le milieu, le matériau, la base et la matière minérale, un agent de cohésion ou texture. Cet agent est de préférence un liant notamment métallique tel que calcium, magnésium, silicium, baryum, sodium, fluor, aluminium, fer, manganèse, zinc, ou organique tel que collagène, muco-polysaccharide et composé poly-cellulosique.

On comprend déjà que cet agent fait éventuellement office de composé d'inactivation, dans le cas d'une base comportant une structure vivante, cultivée ou collectée. Avantageusement, les proportions et la composition de l'agent de cohésion et/ou de texture précité sont choisies pour que la matière finale présente une structure, une dureté ou élasticité prédéterminée.

Durant l'une des phases ou étapes du procédé, un additif par exemple moussant, fibreux, agglomérant, peut être incorporé à la base et/ou à la matière minérale précitées.

Par ailleurs, le procédé comprend une phase de déshydratation au moins partielle d'un ou plusieurs composants de la base et/ou de la matière biominérale. Cette phase de déshydratation peut être au moins partiellement effectuée par filtrage, irradiation, centrifugation, traitement thermique et/ou cryogénique.

Il est envisageable suivant le procédé, que les phases d'inactivation, déshydratation, filature et tissage, soient au moins partiellement effectuées simultanément.

L'étape de traitement peut comprendre des phases de conditionnement de la matière biominérale sous forme de fils aptes au tissage.

Un exemple de déroulement des phases du procédé est donné ci-après :
la base est constituée par une biomasse obtenue par culture in vitro (sur supports nutritifs variés, soit solides, rigides, semi-rigides ou souples, soit encore liquides), conjointe d'un micro-organisme minéralisable tel un mycélium d'ascomycète du genre tuber et d'une bactérie minéralisatrice tel pseudomonas fluorescens ou pseudomonas putida ou bacillus species ou buttauxiella agrestis ou serratia marcescens ou rhodococcus.
   La biomasse peut être obtenue par culture de diatomophycées.

Les trames minéralisées obtenues constituent des membranes biominérales. La nature biochimique de tous les produits obtenus par le procédé est celles des fibres naturelles, protéiques, cellulosiques ou ligneuses.

Le procédé de fabrication ou d'obtention d'une matière ou composition minérale consiste à :
- Préparer une base comprenant une quantité prédéterminée d'un matériau minéral, synthétisé par au moins une ou partie de structure vivante choisie parmi le règne végétal, animal et/ou les micro-organismes ; et
- Traiter ladite base de façon à la transformer en une matière minérale inactivée et de texture prédéfinie en fonction d'un usage extemporané choisi, et par exemple en tant que charge d'apport, isolant thermique, phonique, filtre, membrane acoustique, paroi décoratrice, tissu, fil, film, substance nutritive pour les sols, ou substance à usage médical et/ou paramédical déterminée.

D'une part, l'étape de préparation de la base comporte une phase consistant à cultiver la structure vivante précitée durant une période et dans un milieu tels qu'au moins une partie dite 〈〈 biomasse 〉〉 dudit matériau est alors produite ou synthétisée par cette structure.

Cette étape de préparation est réalisée par exemple in situ par contact avec des micro-organismes et/ou des organismes minéralisateurs choisis dans un environnement adéquat.

D'autre part, l'étape de traitement comprend une phase d'inactivation de la structure vivante effectuée par adjonction d'au moins un sel tel que par exemple oxyde de magnésium, sulfate de magnésium, chlorure de calcium, chlorure de baryum, sous forme anhydre de préférence.

Cette phase d'inactivation est exécutée suivant une ou plusieurs méthodes, seules ou en complément, dont l'irradiation, la déshydratation et/ou l'élévation à haute température.

De préférence, la structure vivante est sélectionnée pour que la matière minérale contienne au moins l'un des constituants suivants : précipités calciques, siliciques, fluorés, barytés, ferrugineux et autres.

Le procédé selon l'invention permet en outre d'obtenir un substrat susceptible d'être inactivé, à usage extemporané choisi, notamment comme isolant thermique, phonique, filtre, membrane acoustique, paroi décoratrice, tissu, fil, ou film.

Ce substrat peut être sous forme membranaire, souple ou solide, plus ou moins cristallisée, d'épaisseur variable, ou sous forme pulvérulente ou granuleuse, ou encore sous forme fluide, gazeuse ou liquide.

L'invention permet également d'obtenir une substance minérale nutritive inactivée à usage extemporané, par exemple en tant que charge d'apport, notamment à tout type de sol naturel et/ou artificiel, ou encore toute composition du type engrais, terreau ou amendements divers.

Pour cela, l'étape de préparation de la base comporte une phase consistant à doser celle-ci en éléments majeurs ou oligo-éléments, suivant leur insuffisance dans les sols et suivant les besoins des cultures.

## Revendications

1. Procédé de fabrication ou obtention d'une matière ou composition minérale et caractérisé par les étapes consistant à :
- préparer une base comprenant une quantité prédéterminée d'un matériau minéral ou biominéral synthétisé par au moins une ou partie de structure vivante choisie parmi le règne végétal, animal et/ou les micro-organismes, ladite structure vivante étant cultivée durant une période et dans un milieu tels qu'au moins une partie dite 〈〈biomasse minérale〉〉 dudit matériau est alors produite ou synthétisée par celle structure; et
- traiter ladite base de façon à la transformer en une matière minérale ou biominérale inactivée, notamment par une phase d'inactivation de la structure vivante, et de texture prédéfinie en fonction d'un usage extemporané choisi, par exemple en tant que charge d'apport, isolant thermique ou phonique, filtre, membrane acoustique, paroi décoratrice, tissu, fil, film, substance nutritive pour sols, substance à usage médical et/ou paramédical.

2. Procédé selon la revendication 1, caractérisé en ce que-la phase d'inactivation est effectuée par adjonction d'au moins un sel tel que par exemple l'oxyde de magnésium, le sulfate de magnésium, le chlorure de calcium, le chlorure de baryum, le fluosilicate de sodium ou le silicate de sodium, sous forme anhydre de préférence.

3. Procédé selon l'une des revendications 1 ou 2, caractérisé en ce que la phase d'inactivation est exécutée suivant une ou plusieurs méthodes, seules ou en complément, dont l'irradiation, la déshydratation et/ou l'élévation à haute température.

4. Procédé selon l'une des revendications précédentes, caractérisé en ce que la structure vivante est sélectionnée pour que la matière minérale contienne au moins l'un des constituants suivants : précipités calciques, siliciques, fluorés, barytés, ferrugineux et autres.

5. Procédé selon l'une des revendications précédentes, caractérisé en ce que l'étape de préparation est réalisée in situ, par contact avec des micro-organismes et/ou des organismes minéralisateurs choisis dans un environnement adéquat.

6. Substrat susceptible d'être inactivé obtenu suivant le procédé selon l'une des revendications précédentes, à usage extemporané choisi, notamment comme isolant thermique, phonique, filtre, membrane acoustique, paroi décoratrice, tissu, fil, film.

7. Substrat selon la revendication 6, caractérisé en ce qu'il est sous forme membranaire, souple ou solide, plus ou moins cristallisée, d'épaisseur variable, ou sous forme pulvérulente ou granuleuse, ou encore sous forme fluide, gazeuse ou liquide.

8. Procédé selon la revendication 1, de production d'une substance nutritive pour sols, naturels et/ou artificiels, caractérisé en ce que l'étape de préparation de la base comporte une phase consistant à doser celle-ci en éléments majeurs ou oligo-éléments suivant leurs insuffisances dans les sols et suivant les besoins des cultures.

9. Substance minérale nutritive inactivée obtenue suivant le procédé selon la revendication 8, à usage extemporané, par exemple en tant que charge d'apport, notamment à tout type de sol naturel et/ou artificiel, ou encore toute composition du type engrais, terreau ou amendements divers.

## Claims

1. Process for making or obtaining a mineral matter or composition and characterized by the steps consisting in:
- preparing a base comprising a predetermined quantity of a mineral or biomineral material synthesized by at least one or part of living structure chosen from the vegetable, animal kingdom and/or micro-organisms, said living structure being cultivated for a period of time and in a medium such that at least a part of said material, so-called "mineral biomass", is then produced or synthesized by this structure; and
- treating said base so as to transform it into an inactivated mineral or biomineral matter, in particular by a phase of inactivation of the living structure, and of texture predefined as a function of a chosen extemporaneous use, for example as additional filler, heat- or sound insulating agent, filter, acoustic membrane, decorating wall, woven fabric, yarn, film, nutritive substance for soils, substance for medical and/or paramedical use.

2. Process according to Claim 1, characterized in that the phase of inactivation is effected by addition of at least one salt such as for example magnesium oxide, magnesium sulphate, calcium chloride, barium chloride, sodium fluosilicate or sodium silicate, preferably in anhydrous form.

3. Process according to one of Claims 1 or 2, characterized in that the phase of inactivation is effected in accordance with one or more methods, alone or complementary, including irradiation, dehydration and/or raising to high temperature.

4. Process according to one of the preceding Claims, characterized in that the living structure is selected so that the mineral matter contains at least one of the following constitutes: calcic, silicic, fluoric, barium, ferruginous and other precipitates.

5. Process according to one of the preceding Claims, characterized in that the step of preparation is effected in situ, by contact with micro-organisms and/or mineralizer organisms chosen in an adequate environment.

6. Substrate capable of being inactivated obtained in accordance with the process according to one of the preceding Claims, for chosen extemporaneous use, in particular as heat-, sound insulating agent, filter, acoustic membrane, decorating wall, woven fabric, yarn, film.

7. Substrate according to Claim 6, characterized in that it is in membrane form, supple or solid, more or less crystallized, of variable thickness, or in pulverulent or granulous form, or in fluid, gaseous or liquid form.

8. Process according to Claim 1 for producing a nutritive substance for natural and/or artificial soils, characterized in that the step of preparation of the base comprises a phase consisting in dosing the latter with major elements or oligo-elements as a function of the insufficiency thereof in the soils and depending on the needs of the crops.

9. Inactivated nutritive mineral substance obtained according to the process of Claim 8, for extemporaneous use, for example as additional filler, particularly for any type of natural and/or artificial soil, or any composition of fertilizer type, compost or various enriching agents.

## Patentansprüche

1. Verfahren zur Herstellung oder Gewinnung eines mineralischen Stoffes oder Zusammensetzung, **gekennzeichnet** durch die folgenden Schritte:
- Herstellen eines Grundstoffes, der eine vorbestimmte Menge eines mineralischen oder biomineralischen Materials aufweist, das durch wenigstens eine aus dem Reich der Pflanzen, Tiere und/oder Mikroorganismen gewählte lebende Struktur oder deren Teil synthetisiert ist, wobei die lebende Struktur für einen Zeitraum in einer Umgebung kultiviert wird, so dass wenigstens ein Teil der 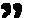 mineralischen Biomasse" des Materials durch diese Struktur produziert oder hergestellt wird; und
- Behandeln des Grundstoffes zu dessen Umwandlung in einen mineralischen oder biomineralischen Stoff, insbesondere durch eine Phase der Inaktivierung der lebenden Struktur und der vorbestimmten Textur in Abhängigkeit der gewählten Sofortverwendung, zum Beispiel als Zuführstoff, Wärme- oder Schallisolator, Filter, akustische Membran, Dekorationsmembran, Gewebe, Faden, Film, Nährsubstanz für Böden, Substanz zur Verwendung in der Medizin oder im medizinischen Hilfsbereich.

2. Verfahren nach Anspruch 1, **dadurch gekennzeichnet, dass** die Phase der Inaktivierung durch Zusatz wenigstens eines Salzes wie zum Beispiel Magnesiumoxid, Magnesiumsulfat, Kalziumchlorid, Bariumchlorid, Natriumfluorsilikat oder Natriumsilikat, vorzugsweise in der Form als Anhydrid erfolgt.

3. Verfahren nach einem der Ansprüche 1 oder 2, **dadurch gekennzeichnet, dass** die Phase der Inaktivierung entsprechend einer oder mehrerer Methoden, alleine oder in Ergänzung, wie Bestrahlung, Dehydratation und/oder Erwärmung auf hohe Temperatur, ausgeführt wird.

4. Verfahren nach einem der vorhergehenden Ansprache, **dadurch gekennzeichnet, dass** die lebende Struktur so gewählt ist, dass der mineralische Stoff wenigstens einen der folgenden Bestandteile enthält: Kalzium, Kieselsäure, Baryt, Eisen und anderes enthaltender Niederschlag.

5. Verfahren nach einem der vorhergehenden Ansprüche, **dadurch gekennzeichnet, dass** der Schritt der Herstellung in situ durch Kontakt mit Mikroorganismen und/oder mineralbildenden Organismen, die in einer geeigneten Umgebung ausgewählt sind, erfolgt.

6. Substrat welches geeignet ist inaktiviert zu werden und entsprechend dem Verfahren nach einem der vorhergehenden Ansprüche erhalten wird, zur späteren ausgewählten Verwendung, insbesondere als Wärme-, Schallisolator, Filter, akustische Membran, Dekorationswand, Gewebe, Faden, Film.

7. Substrat nach Anspruch 6, **dadurch gekennzeichnet, dass** es in membranartiger, nachgiebiger oder fester, mehr oder weniger kristallisierter Form mit unterschiedlicher Dicke, oder in pulverisierter oder granulierter Form, oder in fluider, gasförmiger oder flüssiger Form vorliegt.

8. Verfahren nach Anspruch 1 zur Herstellung einer Nährsubstanz für natürliche und/oder künstliche Böden, **dadurch gekennzeichnet, dass** der Schritt zur Herstellung des Grundstoffes eine Phase umfasst; die darin besteht; diesen den Mängeln der Böden und den Erfordernissen der Kulturen entsprechend in größere Elemente oder Oligo-Elemente zu dosieren.

9. Inaktivierte mineralische Nährsubstanz, welche entsprechend dem Verfahren nach Anspruch 8 erhalten wurde, zur späteren Verwendung zum Beispiel als Zuführstoff, insbesondere für jede Art eines natürlichen und/oder künstlichen Bodens, oder jede Zusammensetzung vom Typ eines Düngers, eines humusreichen Bodens oder für unterschiedliche Verbesserungsmittel.
